# EUROPEAN PATENT APPLICATION

(11) **EP 3 922 730 A1**
(43) Date of publication of application: **15.12.2021**
(21) Application number: 20752573.4
(22) Date of filing: 07.02.2020
(51) Int. Cl.: C12Q 1/6809, C12Q 1/6869, G16B 25/00

(54) **METHOD FOR ANALYZING MRNA PRECURSOR, INFORMATION PROCESSING APPARATUS, AND COMPUTER PROGRAM**

(30) Priority: 08.02.2019 JP 2019021463; 29.11.2019 JP 2019217279
(71) Applicant: Zenick Corporation, Tokyo, 104-0045 (JP); Okada, Norihiro, Tokyo, 157-0066 (JP)
(72) Inventor: SUGINO Toru, Tokyo 104-0045 (JP); OKADA Norihiro, Tokyo 157-0066 (JP)
(74) Representative: Manitz Finsterwald Patent- und Rechtsanwaltspartnerschaft mbB
(86) International application number: PCT/JP2020/004901
(87) International publication number: WO 2020/162613

(57) **Abstract**

Provided is a technology involving analyzing the state of mRNA of a eukaryote in a simple manner, and facilitating, for example, the proposal of a measure in the event that the state is an unpreferred state. That is, provided is an analysis method for a precursor mRNA, including quantitatively analyzing a removal state of an intron in a precursor mRNA of a eukaryote and outputting information specific to the eukaryote. The presence or absence of an aged-type or presymptomatic disease-type splicing pattern different from a healthy-type splicing pattern is detected from a quantitatively analyzed removal state of the intron (Step S3). From a pre-made substance list, information on a pharmaceutically acceptable substance for bringing the aged-type or presymptomatic disease-type splicing pattern closer to the healthy-type splicing pattern is identified (Step S5). The identified information is output as information specific to the eukaryote serving as an analysis object (Step S6).

## Description

### Technical Field

The present invention relates to a technology for analyzing biological information, such as a technology for analyzing a human body state through analysis of an mRNA in a eukaryote, or a technology for analyzing a human body state through analysis of a gene.

### Background Art

Hitherto, there has been known, for example, a technology for analyzing a human body state through structural analysis of a gene. For example, in Patent Literature 1, there is a disclosure of a technique for analyzing an association between a genetic abnormality and a disease. As a cause of a genetic abnormality, there are known, for example, a mutation occurring in a coding region, and a failure in a transcription/translation process.

A description is made by taking eukaryotes, including humans, as an example. A cell nucleus of the eukaryote has all of its genetic information in DNA. An individual gene is transcribed as required, to produce a precursor mRNA. The precursor mRNA serves as an origin of a blueprint for a protein to be produced in the body, and contains exons, which are portions containing information for protein synthesis, and an intron present between the exons.

The intron is essentially unnecessary, and hence is removed by splicing, which is a molecular editing process, and then the exons are joined together to thus complete an mRNA (mature mRNA) serving as the blueprint for the protein to be produced in the body. The mRNA is transported to the cytoplasm and translated to produce the protein. It is said that, when there is some abnormality in the splicing, the intron is not sufficiently removed, and hence a state unpreferred for the eukaryote, such as a state of aging or disease, is liable to occur.

### Citation List

### Patent Literature

[PTL 1] JP 2002-223760 A

### Summary of Invention

### Technical Problem

Splicing regulators are involved in the splicing of a precursor mRNA. In addition, the splicing regulators are diverse in kind, and also vary on an individual basis among eukaryotes. Accordingly, it is difficult to rapidly analyze the state of mRNA in an individual eukaryote, and there is no disclosure thereof in Patent Literature 1.

In addition, no investigation has yet been made of a measure specific to a eukaryote in the event that the mRNA state is a state unpreferred for the eukaryote.

The present invention has been made under the above-mentioned background, and a primary object of the present invention is to provide a technology for analyzing a biological state by analyzing a biosignal, or mRNA or DNA.

Another object of the present invention is to provide a technology involving analyzing the state of mRNA in a simple manner, and facilitating, for example, the proposal of a measure in the event that the state is an unpreferred state.

### Solution to Problem

In order to achieve the above-mentioned objects, according to one embodiment, there is provided an analysis method for a precursor mRNA, including: quantitatively analyzing a removal state of an intron in a precursor mRNA of a eukaryote, to thereby detect the presence or absence of a second splicing pattern having a possibility of deriving a gene expression pattern different from that of a first splicing pattern that derives a gene expression pattern serving as a reference; and when the second splicing pattern is present, identifying, from a pre-made substance list, information on a pharmaceutically acceptable substance for bringing the second splicing pattern closer to the first splicing pattern, followed by outputting the identified information as information specific to the eukaryote.

### Advantageous Effects of Invention

According to the present invention, the technology for analyzing a biological state by analyzing a biosignal, or mRNA or DNA can be provided. In addition, it is also possible to analyze the state of mRNA in a eukaryote in a simple manner, and to facilitate, for example, the proposal of a measure in the event that the state is an unpreferred state.

### Brief Description of Drawings

FIG. 1 is an explanatory diagram of alternative splicing.
FIG. 2 is an explanatory diagram of IJC and SJC.
FIG. 3(a) to FIG. 3(c) are explanatory diagrams of intron retention in a Sirt7 gene.
FIG. 4(a) to FIG. 4(c) are explanatory diagrams of intron retention in a Cyp27a1 gene.
FIG. 5(a) to FIG. 5(c) are explanatory diagrams of intron retention in a Ppard gene.
FIG. 6(a) to FIG. 6(c) are explanatory diagrams of intron retention in an Acadm gene.
FIG. 7(a) to FIG. 7(c) are explanatory diagrams of intron retention in a Decr2 gene.
FIG. 8 is an explanatory diagram of the results of recovery of intron retention.
FIG. 9 is an explanatory diagram of a cluster of 368 loci in genes in the liver and driver genes.
FIG. 10 is a hardware configuration diagram of an information processing apparatus according to an embodiment of the present invention.
FIG. 11 is a functional block configuration diagram of the information processing apparatus.
FIG. 12 is an explanatory diagram of the processing procedure of an analysis method performed with the information processing apparatus.
FIG. 13(a) to FIG. 13(c) are explanatory diagrams of SE splicing patterns in a Ptbp1 gene.
FIG. 14 is an explanatory diagram of the concept of presymptomatic disease.
FIG. 15(A) to FIG. 15(D) are graphs showing the results of measurement of characteristics of introns.

### Description of Embodiments

Embodiments of the present invention are described below with reference to the drawings.

### <First Embodiment>

The inventors of the present invention have experimented with analysis of mRNA in a eukaryote and a remedial measure (recovery of a living body from an undesired state, such as aging) for a case in which the state of mRNA is unpreferred (e.g., a case in which the state of mRNA exhibits aging or any other undesired biological state).

In the first embodiment, an overview of the experiment and the results thereof are described. In this experiment, attention was focused on aging as an example in which the biological state of the eukaryote is an undesired state. However, the present invention is not limited to aging, and is also widely applicable to other biological states or human body states, such as a presymptomatic disease state described in a second embodiment.

In the first embodiment, an aging model mouse called a klotho mouse, and a wild-type mouse (Wild type mouse) serving as a comparative example were used as examples of the eukaryote. The klotho mouse is also called a human premature aging syndrome mouse, and ages faster than the wild-type mouse. The klotho mouse has an average life span of about 60 days, and enables an experiment to be performed within a shorter period of time than the wild-type mouse. For this and other reasons, the klotho mouse is used in various experiments as a substitute for a human. When those mice do not particularly need to be distinguished from each other, the mice are referred to as "subjects". In addition, as an example of a measure for changing a state of mRNA in a subject unpreferred for the eukaryote so as to achieve recovery, the subject was allowed to ingest a pharmaceutically acceptable substance.

The pharmaceutically acceptable substance is a substance that does not cause, for example, excessive toxicity, stimulation, anaphylaxis, immunogenicity, or any other problem or complication in humans, mice, or the like. Examples of such substances include: a substance itself capable of being ingested by humans, mice, or the like; a liquid substance obtained by dissolving the substance in a liquid; and a product obtained by making the substance ingestible as a formulation. In this experiment, a Kampo medicine, which is a Japanese traditional medicine, was used as an example. It is considered that the Kampo medicine exhibits a clinical effect through a synergistic effect caused by a combination of thousands of chemical substances contained in its many herbal ingredients. However, there is no known research in which the action mechanism of the Kampo medicine is elucidated from a molecular biological point of view. In view of this, the inventors of the present invention allowed subjects to ingest the Kampo medicine, and investigated its effect on splicing in organs of the subjects through known RNA-seq analysis. Specifically, the subjects were allowed to ingest the Kampo medicine, and then differences in splicing including the removal states of introns in precursor mRNAs of the subjects were analyzed. Through this analysis, the influence of the ingested substance on the subjects was investigated with regard to, for example, an mRNA exhibiting a splicing pattern that derived a gene expression pattern associated with aging (aged-type splicing pattern). In the first embodiment, the aged type is described. However, the splicing pattern is not limited to the aged type, and the present disclosure is also applicable to a presymptomatic disease-type splicing pattern, which is a splicing pattern that derives a gene expression pattern associated with the presymptomatic disease state to be described later.

There are two kinds of genes whose splicing patterns in subjects are changed through the ingestion of the Kampo medicine. One is a gene for a factor involved in splicing directly or incidentally, and at present, a group including 21 genes is known. The other is a gene related to mitochondria in an organ, or lipid or glucose metabolism. At present, a group including about 350 genes is known.

The former genes dominate the latter group of genes through regulation of splicing, and hence are called "driver genes" or "upstream genes". Meanwhile, the latter group of genes are called "target genes" or "downstream genes" in relation to the "upstream genes". The upstream genes gradually change their splicing patterns to the aged type along with, for example, aging, and the splicing patterns of the genes downstream thereof also age inevitably. Accordingly, the gene whose splicing pattern is changed is desirably an upstream gene.

The inventors of the present invention have verified that the herbal ingredients of the Kampo medicine act on the above-mentioned transcription or epigenome system in a subject to change (recover) the splicing pattern of the precursor mRNA in an upstream gene to its state before aging. Herein, for convenience of description, the state before aging in a subject is referred to as "healthy state" as an example of a state before the occurrence of an unpreferred state. In addition, a gene or a splicing pattern in an aged state is referred to as "aged type", and a gene or a splicing pattern in a healthy state is referred to as "healthy type".

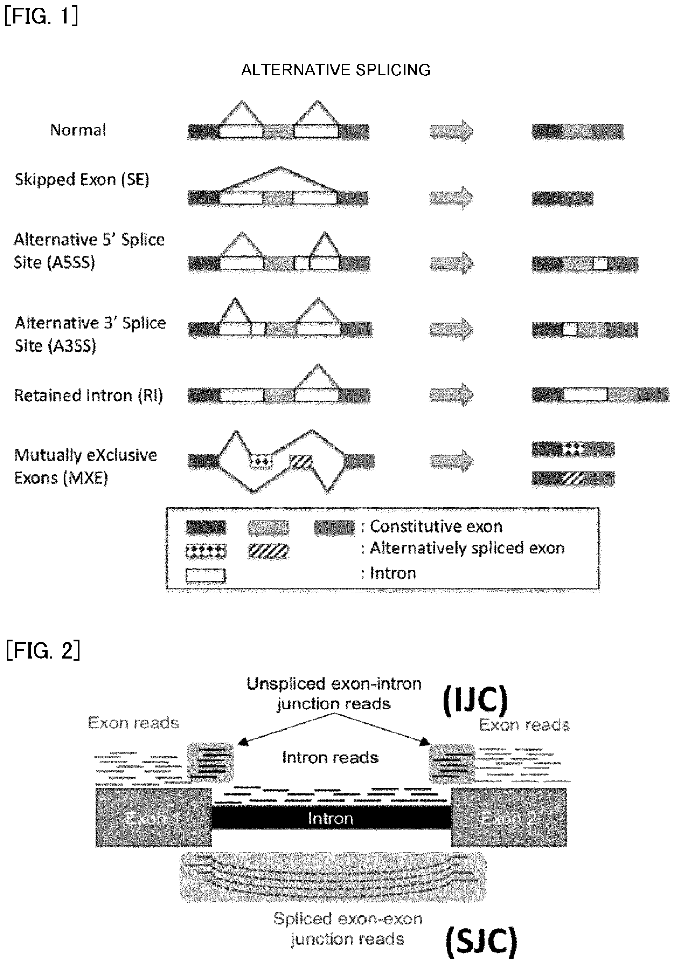

Now, as an assumption for the experiment, the splicing of a precursor mRNA that occurs in common among eukaryotes including the klotho mouse is described. FIG. 1 is an explanatory diagram of alternative splicing, which results in various splicing patterns. Alternative splicing is a process of joining exons together in six kinds of patterns with the above-mentioned splicing regulators.

In FIG. 1, there are illustrated exons to be invariably used (constitutive exons), alternatively spliced exons, and introns to be normally removed. In a Normal pattern illustrated in the uppermost row of FIG. 1, an intron is present between the leading constitutive exon and the second constitutive exon, and an intron is also present between the second constitutive exon and the third constitutive exon. In the Normal pattern, those two introns are removed, and the three constitutive exons are connected together. This state is the most common splicing pattern.

In a Skipped Exon (SE) pattern illustrated in the second row of FIG. 1, the leading constitutive exon and the third constitutive exon are connected together, and the introns therebetween and the second constitutive exon are removed.

In an Alternative 5' Splice Site (A5SS) pattern illustrated in the third row of FIG. 1, part of the intron between the second constitutive exon and the third constitutive exon remains instead of being removed. Accordingly, after splicing, part of the intron between the second constitutive exon and the third constitutive exon is left.

In an Alternative 3' Splice Site (A3SS) pattern illustrated in the fourth row of FIG. 1, part of the intron between the leading constitutive exon and the second constitutive exon remains instead of being removed. Accordingly, after splicing, part of the intron between the leading constitutive exon and the second constitutive exon is left.

In a Retained Intron (RI) pattern illustrated in the fifth row of FIG. 1, the intron between the leading constitutive exon and the second constitutive exon is not removed, and only the intron between the second constitutive exon and the third constitutive exon is removed.

In Mutually eXclusive Exons (MXE) illustrated in the sixth row of FIG. 1, before splicing, the leading constitutive exon is connected to and followed by a first alternatively spliced exon, a second alternatively spliced exon, and the second constitutive exon, and after splicing, the following two mRNAs are produced: an mRNA in which the leading constitutive exon, the first alternatively spliced exon, and the second constitutive exon are connected together; and an mRNA in which the leading constitutive exon, the second alternatively spliced exon, and the second constitutive exon are connected together.

In this experiment, how alternative splicing in a precursor mRNA was performed was analyzed by a known RNA-seq method (RNA-seq analysis). The analysis of splicing may be performed using "rMATS", which is well-known software in the art of bioinformatics. rMATS allows operation as visualization means configured to draw detection results so as to be shown on a display through use of the known R language. The R language is an open source/free software programming language for statistical analysis and a development and execution environment therefor, and can be easily utilized in accordance with intended use.

In addition, on the basis of the results of the RNA-seq analysis, a relationship between the splicing pattern of the precursor mRNA and aging was analyzed, and the possibility of recovery of a changed splicing pattern was searched for.

In this procedure, for the purpose of investigating the splicing pattern, an intron including junction (IJC) and a skipping junction after the splicing of the precursor mRNA were defined as described below in this experiment.
IJC: an exon junction after precursor mRNA splicing in which an intron is present between exons
SJC: an exon junction after precursor mRNA splicing in which an intron between exons is removed

A conceptual diagram of IJC and SJC is illustrated in FIG. 2. In the lower part of FIG. 2, it is illustrated that the introns are completely removed and the exons are joined together. This state corresponds to the Normal pattern in the uppermost row of FIG. 1, and is a healthy state. However, in the case of an aged subject, the introns are not sufficiently removed in splicing, with the result that the IJC in the mRNA is increased as compared to that of a subject in a healthy state while the SJC is decreased. This is caused by portions straddling the exon regions and the intron region in FIG. 2. Those portions are not recognized as introns, and hence are not removed, consequently remaining in the mRNAs.

In view of the foregoing, log₂IJC/SJC is calculated as an indicator showing to what extent the introns are removed in alternative splicing. This is an indicator (fold change) showing how high the IJC is with respect to the SJC. This value is a value obtained by taking the log of each of the values of the IJC and the SJC with base 2 (log₂IJC and log₂SJC), followed by subtraction. This value serves as an indicator of the degree of retention of introns at the boundary between exons and introns.

That is, the mRNA of an aged subject shows an increase in log₂IJC/SJC as compared to a control. This value can be used as an indicator of aging. A few test examples assuming the foregoing are described.

### [Test Example 1]

In Test Example 1, the influence on alternative splicing (splicing patterns resulting therefrom) exhibited when subjects were allowed to ingest Juzen-taiho-to as a Kampo medicine was analyzed on the basis of specimen data acquired from the subjects. Juzen-taiho-to is a blend of ten kinds of crude drugs, and is known as a Kampo medicine to be prescribed for anorexia, fatigue, decreased physical strength after illness, and the like. The Juzen-taiho-to used was "TSUMURA Juzentaihoto Extract Granules for Ethical Use" manufactured by Tsumura & Co. Its ingredients are as shown below. The number accompanying each ingredient represents its weight ratio (relative ratio of its weight).
Astragalus root 3.0
Ginseng 3.0
Cinnamon bark 3.0
Japanese angelica root 3.0
Cnidium rhizome 3.0
Peony root 3.0
Rehmannia root 3.0
Atractylodes lancea rhizome 3.0
Poria sclerotium 3.0
Glycyrrhiza 1.5

In order to determine the influence on alternative splicing, a plurality of klotho mice and a plurality of wild-type mice serving as subjects were allowed to ingest Juzen-taiho-to at the same ratio in various environments.

In Test Example 1, as described below, subjects of a first group to a fourth group were fed over 7 weeks, and splicing patterns at the production of mRNAs were analyzed for organs, namely the heart, the kidneys, and the liver, and blood. An equal amount of Juzen-taiho-to was added to an equal amount of a normal feed. In addition, feeding was separately performed for the case of adding Juzen-taiho-to and the case of not adding Juzen-taiho-to, and thus it was determined whether or not a change in gene expression occurred. For convenience of description, the feed without added Juzen-taiho-to is referred to as "normal feed", and the feed with added Juzen-taiho-to is referred to as "additive-containing feed".

First group: Five klotho mice were fed with the normal feed over 3.5 weeks, and then fed with the additive-containing feed over 3.5 weeks.

Second group: Five klotho mice were fed with the normal feed over 3.5 weeks, and fed with the normal feed for the next 3.5 weeks as well.

Third group: Five wild-type mice were fed with the normal feed over 3.5 weeks, and then fed with the additive-containing feed used in the first group over 3.5 weeks.

Fourth group: Five wild-type mice were fed with the normal feed over 4 weeks, and fed with the normal feed for the next 4 weeks as well.

The subjects of the first to fourth groups were kept in the same environment. After the completion of the keeping period, specimen data on organs, namely the liver, the kidneys, and the heart, and analysis data on blood were acquired from each of the subjects, and each set of specimen data was subjected to RNA-seq analysis to detect a splicing pattern (gene expression amount). As a result, for the organs, namely the liver, the kidneys, and the heart, and the blood, in a comparison between the klotho mice of the first group fed with the additive-containing feed and the klotho mice of the second group fed with the normal feed, it was recognized that 702 genes were changed in their splicing patterns resulting from alternative splicing of their precursor mRNAs. Of the 702 changed genes, 27 genes are upstream genes of splicing, and dominate many downstream genes. It is evident that the changes in those 27 genes and changes in the splicing patterns in which the genes are involved are deeply involved in the aging of the organs.

In addition, in the klotho mice of the first group continuously fed with the additive-containing feed, it was found that the splicing patterns of a larger number of upstream genes were recovered to healthy types.

In particular, in the genes in the liver, a large number of changes in alternative splicing (changes in splicing patterns) were observed in 368 loci between the klotho mice repeatedly fed with the additive-containing feed and the klotho mice fed only with the normal feed, and 88.3% of the changes (325 loci) were each a change from an aged type to a healthy type. That is, the recovery of a splicing pattern from an aged type to a healthy type was found.

In addition, the recovery of the splicing pattern was found in 97.2% of mRNAs in which there occurred, among the differences in alternative splicing, in particular, a phenomenon called intron retention (RI), in which exons were joined while an intron to be normally removed remained.

Now, in order to investigate in more detail the recovered state for such mRNAs, analysis results for a known Sirt7 gene serving as one of the upstream genes are described. It is known that the Sirt7 gene inhibits the activity of myc and suppresses an ER stress, to thereby prevent a fatty liver (Cell reports, 2013). In addition, it is known that the Sirt7 gene controls fatty acid metabolism in the liver via a ubiquitin proteome (Cell Metabol. 2014).

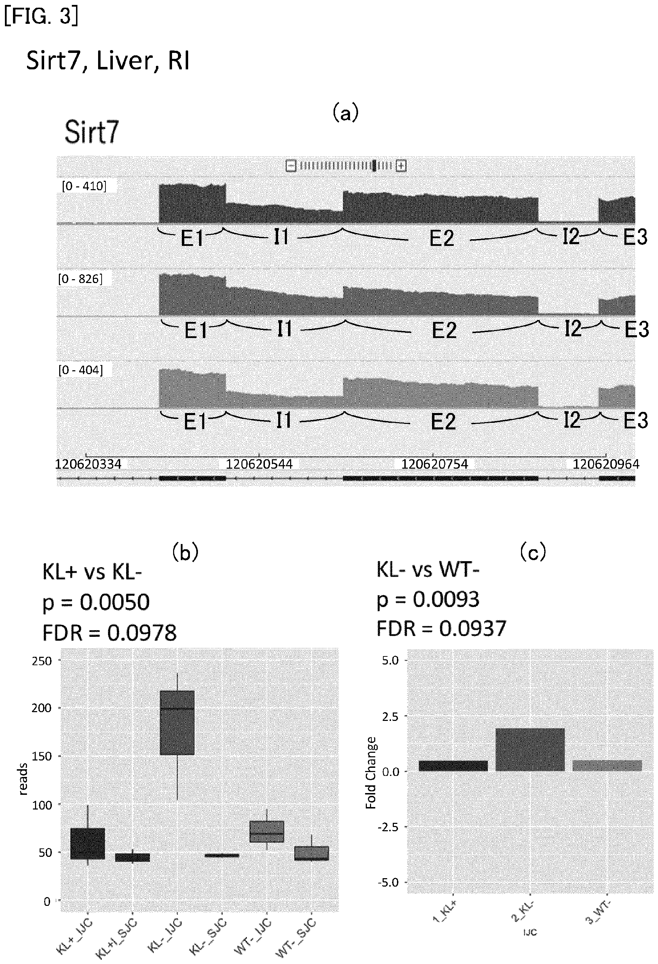

FIG. 3(a) to FIG. 3(c) are explanatory diagrams of intron retention in the Sirt7 gene. In FIG. 3(a), the graph in the uppermost panel is a graph quantifying introns and exons in the liver of the first group, that is, the klotho mice fed with Juzen-taiho-to (abbreviated as KL+). The horizontal axis represents a gene sequence, and the vertical axis represents a Read value. The same applies to the other graphs showing introns and exons.

In the graph in the lowermost panel of FIG. 3(a), E1, E2, and E3 each represent an exon, and I1 and 12 each represent an intron. The same applies to the graphs in the second panel and the third panel. In each of those graphs, the value for intron I1 on the vertical axis is smaller than the values for exons E1, E2, and E3 on the vertical axis, and the exons and the introns can be distinguished quantitatively and visually in each of the graphs. In particular, intron 12 has a value of nearly 0 on the vertical axis, and can be clearly distinguished from exons E1, E2, and E3. This shows the introns and exons in the gene in the liver of the wild-type mice of the fourth group (normal feed only) (abbreviated as WT-).

The graph in the second panel of FIG. 3(a) shows the introns and exons in the gene in the liver of the klotho mice of the second group (abbreviated as KL-). In this graph, the values for intron I1 on the vertical axis are generally higher than the values for intron I1 in KL+. The boundary between each of exons E1 and E2, and intron I1 is relatively clear, but intron I1 and exon E2 both tend to decrease from left to right, and are difficult to distinguish from each other.

The graph in the uppermost panel of FIG. 3(a) investigates the effect of addition of Juzen-taiho-to in the pattern of KL+. In this graph, the value for intron I1 on the vertical axis is smaller than the values for exons E1 and E2 on the vertical axis, and in the same manner as shown in the graph in the lowermost panel, the exons and the introns can be clearly distinguished in the pattern of KL+ as well.

FIG. 3(b) shows the read value of the IJC (KL+_IJC) and the read value of the SJC (KL+_SJC) in the klotho mice continuously fed with the additive-containing feed. In addition,

FIG. 3(b) shows the read value of the IJC (KL-_IJC) and the read value of the SJC value (KL-_SJC) in the klotho mice fed only with the normal feed, and the read value of the IJC (WT-_IJC) and the read value of the SJC value (WT-_SJC) in the wild-type mice fed only with the normal feed. In FIG. 3(b), "p" represents a p-value (significance probability), and FDR represents a false discovery rate. As shown in FIG. 3(b), the p-value was 0.0050, and FDR was 0.0978. Those values support that continuous feeding with the additive-containing feed recovered the splicing pattern to a healthy type in a statistically significant manner.

In addition, in a comparison among KL+ fed with Juzen-taiho-to, the klotho mice KL-fed only with the normal feed, and the wild-type mice WT- fed only with the normal feed, the log₂IJC/SJC value of KL- is extremely larger than the log₂IJC/SJC values of WT- and KL+.

Thus, it was revealed that, in an aged subject, the introns were not sufficiently removed in the splicing of the precursor mRNA, resulting in an increase in IJC and a decrease in SJC in the mRNA as compared to a subject of a healthy type. This fact supports that, as shown in FIG. 3(b), the klotho mice continuously fed with the additive-containing feed have an extremely large IJC as compared to the wild-type mice fed only with the normal feed, resulting in advanced aging.

Meanwhile, the log₂IJC/SJC values of the klotho mice KL+ fed with Juzen-taiho-to are extremely small as compared to the log₂IJC/SJC values of the klotho mice KL-, and show a distribution similar to that of the log₂IJC/SJC values of the wild-type mice WT-. Thus, it was revealed that, in the case of the klotho mice continuously fed with the additive-containing feed, the aging of the Sirt7 gene was suppressed, and the log₂IJC/SJC values showed a distribution close to that of normal wild-type mice.

FIG. 3(c) shows fold change values for the klotho mice KL+ and KL-, and the wild-type mice WT-. In FIG. 3(c), a fold change value on the vertical axis represents a log₂IJC/SJC value. Also in FIG. 3(c), the klotho mice KL+ continuously fed with the additive-containing feed have a small distribution of fold change values like the wild-type mice WT-. Meanwhile, the klotho mice KL- fed only with the normal feed have a fold change value about 4 times as high as those of the klotho mice KL+ and the wild-type mice WT-. Accordingly, in the klotho mice KL+ continuously fed with the additive-containing feed, the aging of the gene is suppressed as compared to the klotho mice KL-.

As can be seen from the foregoing, in the klotho mice KL- fed only with the normal feed, the introns remain in a large amount even after splicing, and hence their differences from the exons are vague, with the result that the exons are not properly joined together. In contrast, in each of the wild-type mice WT- and the klotho mice KL+ continuously fed with the additive-containing feed, the differences between the introns and the exons are distinct, and the exons are properly joined together. This fact supports that, even in klotho mice, continuous feeding with the additive-containing feed suppresses the progression of aging, and splicing is performed in the same manner as in wild-type mice.

The finding that advanced aging causes splicing not to be properly performed is mentioned in, for example, "The emerging role of alternative splicing in senescence and aging (Aging Cell, October 2017)." However, there is no document in which a technique for suppressing the progression of aging is quantitatively elucidated. In this experiment, the continuous feeding of subjects with the additive-containing feed has provided the following surprising effect: splicing is kept normal, and as a result, an aged portion can be recovered.

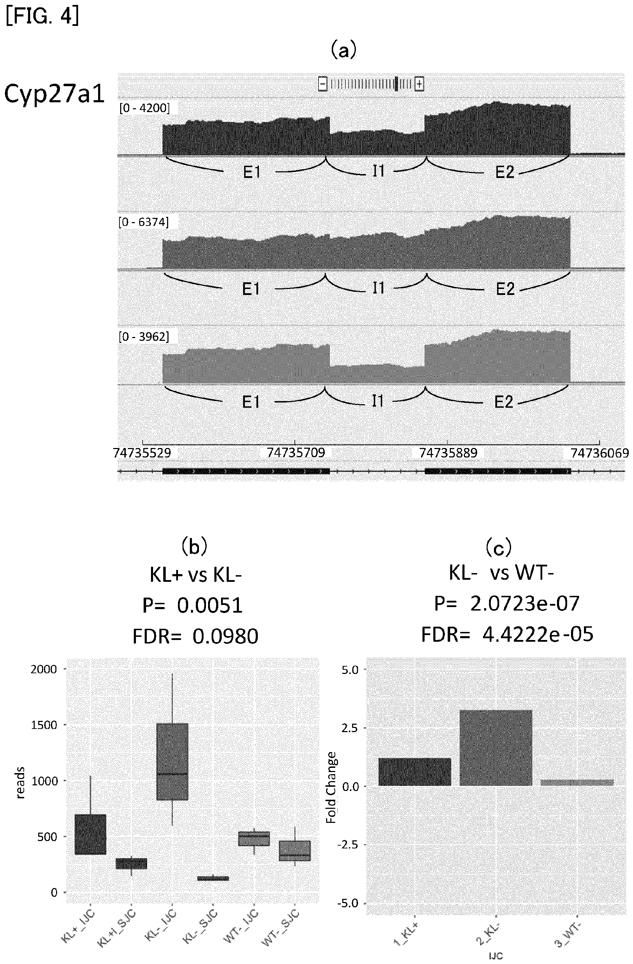

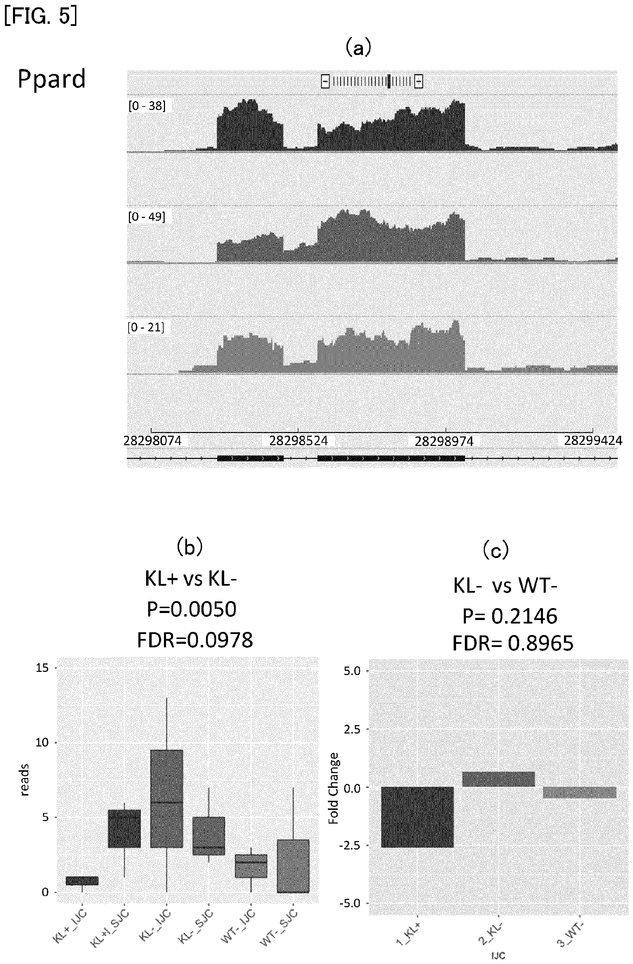

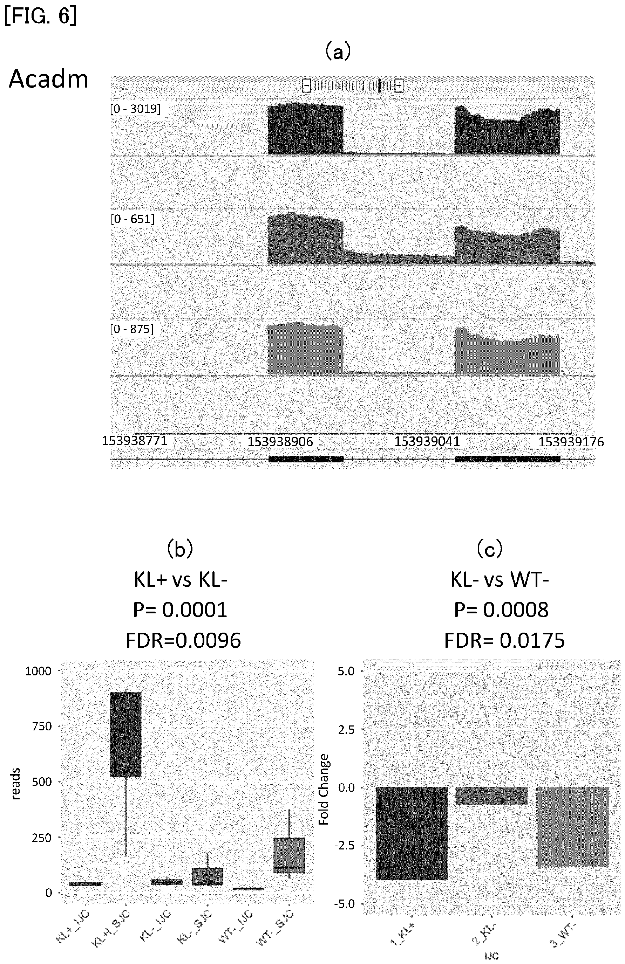

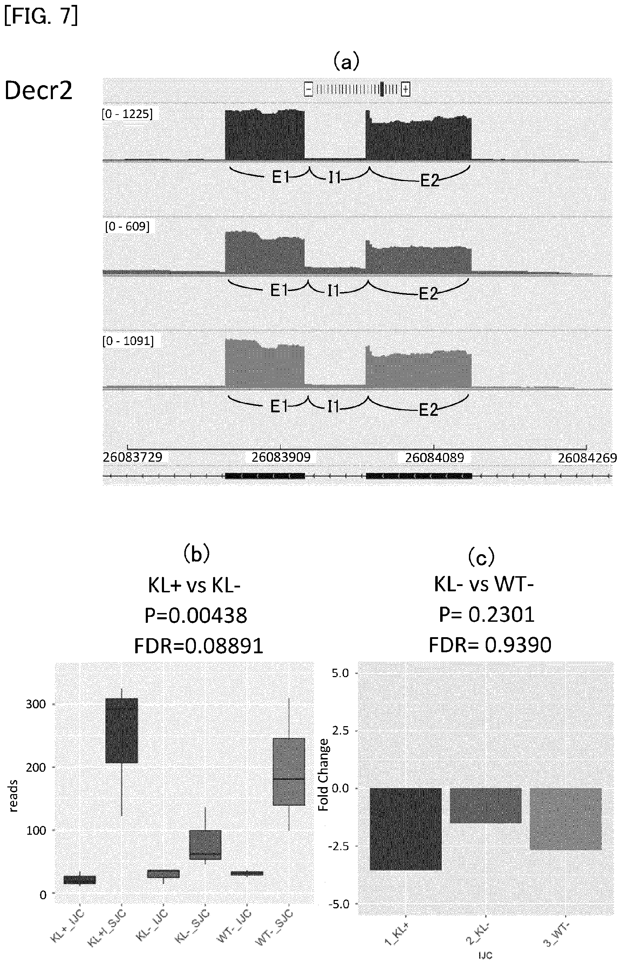

While the analysis results for the Sirt7 gene have been described above, analysis was also performed for genes other than the Sirt7 gene, and hence the results thereof are described. FIG. 4(a) to FIG. 4(c) are explanatory diagrams of intron retention in a Cyp27a1 gene. FIG. 5(a) to FIG. 5(c) are explanatory diagrams of intron retention in a Ppard gene. FIG. 6(a) to FIG. 6(c) are explanatory diagrams of intron retention in an Acadm gene. FIG. 7(a) to FIG. 7(c) are explanatory diagrams of intron retention in a Decr2 gene. In these figures, the vertical axes and horizontal axes of the graphs, and the symbols, such as E1 and I1, each have the same meaning as in FIG. 3(a) to FIG. 3(c).

The Cyp27a1 gene shown in FIG. 4(a) to FIG. 4(c) is a member of the cytochrome P450 superfamily of enzymes. The Cyp27a1 gene is a monooxygenase that catalyzes many reactions involved in drug metabolism and synthesis of cholesterol, steroids, and other lipids, and oxidizes a cholesterol intermediate as part of the bile synthesis pathway.

Also in the case of the Cyp27a1 gene, like the Sirt7 gene shown in FIG. 3(a) to FIG. 3(c), in the klotho mice KL- fed only with the normal feed, the intron remains in a large amount even after splicing, and hence its differences from the exons are vague.

Meanwhile, in the wild-type mice WT- and the klotho mice KL+ continuously fed with the additive-containing feed, the differences between the intron and the exons are distinct, and the exons are properly joined together. In particular, as shown in the second panel of FIG. 4(a), in terms of read value of the IJC of the klotho mice KL- fed only with the normal feed, exon E1, intron I1, and exon E2 show distributions similar to each other, and hence are difficult to distinguish from each other. However, in the graph in the uppermost panel for continuous feeding with the additive-containing feed, as in the graph in the third panel corresponding to the wild-type mice WT-, the intron is clearly distinguished from the exons. That is, the recovery effect of the additive-containing feed on the splicing pattern is even clearer.

This fact supports that, also in the case of the Cyp27a1 gene, even in klotho mice, continuous feeding with the additive-containing feed keeps alternative splicing normal to suppress the progression of aging, and hence splicing is performed in the same manner as in wild-type mice.

FIG. 5(a) to FIG. 5(c) are explanatory diagrams of intron retention in the Ppard gene. The Ppard gene is a member of the peroxisome proliferator-activated receptor (PPAR) family, and regulates the peroxisomal beta-oxidation pathway of fatty acids. As shown in FIG. 5(a) to FIG. 5(c), it is supported that, also in the case of the Ppard gene, even in klotho mice, feeding with Juzen-taiho-to keeps splicing normal to suppress the progression of aging, and hence splicing is performed in the same manner as in normal wild-type mice.

FIG. 6(a) to FIG. 6(c) are explanatory diagrams of intron retention in the Acadm gene. The Acadm gene encodes a medium-chain specific acyl-coenzyme A dehydrogenase, which catalyzes the initial step of the mitochondrial fatty acid beta-oxidation pathway. Defects in the Acadm gene cause a disease characterized by medium-chain acyl-CoA dehydrogenase deficiency, hepatic dysfunction, fasting hypoglycemia, and encephalopathy, which can result in infantile death. As shown in FIG. 6(a) to FIG. 6(c), it is supported that, also in the case of the Acadm gene, even in klotho mice, continuous feeding with the additive-containing feed keeps splicing normal to suppress the progression of aging, and hence splicing is performed in the same manner as in normal wild-type mice.

FIG. 7(a) to FIG. 7(c) are explanatory diagrams of intron retention in the Decr2 gene. The Decr2 gene is an auxiliary enzyme of beta-oxidation, and participates in the degradation of unsaturated fatty acid enoyl-CoA esters having double bonds in both even- and odd-numbered positions in peroxisome. As shown in FIG. 7(a) to FIG. 7(c), it is supported that, also in the case of the Decr2 gene, even in klotho mice, continuous feeding with the additive-containing feed keeps splicing normal to suppress the progression of aging, and hence splicing is performed in the same manner as in normal wild-type mice.

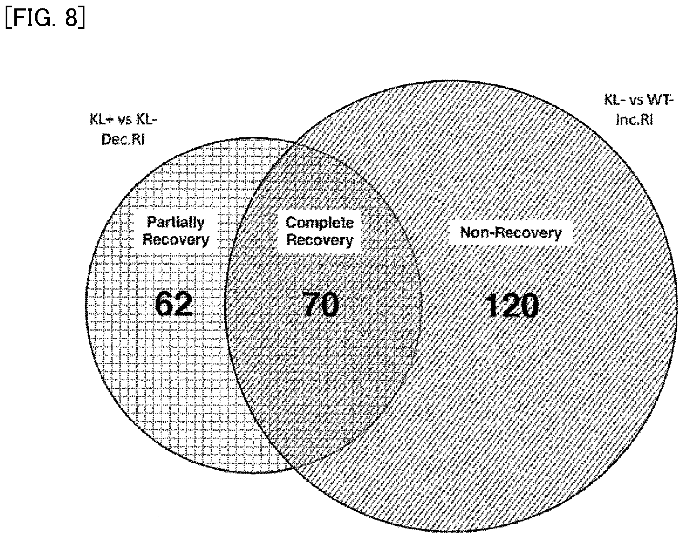

In addition, it was investigated whether each of 252 kinds of genes was recovered from intron retention through administration of JTT. The results are shown in FIG. 8. As shown in FIG. 8, 70 genes underwent complete recovery, 62 genes underwent partial recovery, and 120 genes underwent no recovery (unrecovered aged genes).

In addition, a list of the genes that underwent complete recovery through administration of JTT is shown below. The "chr3:153939069-153939167" written beside Acadm represents its chromosome number in mice and position on the genome. The same applies to the other genes, such as Adck5.
Acadm, chr3:153939069-153939167
Adck5, chr15:76594152-76594447
Adipor1, chr1:134424750-134424922
Aldh4a1, chr4:139642076-139642273
Aldh4a1, chr4:139633896-139633989
Alg6, chr4:99752816-99752885
Anapc5, chr5:122800450-122800593
Anxa11, chr14:25874670-25874784
Atg9a, chr1:75182591-75182737
Atp11b, chr3:35839048-35839214
Bsdc1, chr4:129466822-129466990
Camk1, chr6:113339506-113339581
Cct3, chr3:88300928-88300991
Chkb, chr15:89428713-89428827
Cln3, chr7:126575343-126575412
Cnbp, chr6:87845464-87845557
Ctdsp1, chr1:74393796-74393901
Cyp27a1, chr1:74735863-74736036
Ddx5, chr11:106783958-106784018
Enpp5, chr17:44085204-44086567
Faah, chr4:116000786-116000827
Fastkd2, chr1:63735844-63735968
Ftcd, chr10:76584169-76584299
Gaa, chr11:119274214-119274311
Gatad2b, chr3:90355674-90355785
Galt, chr4:41756681-41756811
Gdi1, chrX:74308129-74308261
Ghdc, chr11:100768217-100768289
Gnb2l1, chr11:48802272-48802420
Gnmt, chr17:46726268-46726411
Hpd, chr5:123181860-123181923
Hsd3b7, chr7:127802785-127803802
Jmjd8, chr17:25830138-25830206
Kctd2, chr11:115430312-115431274
Las1l, chrX:95950336-95950446
Maged1, chrX:94537973-94538065
Med24, chr11:98717909-98718142
Men1, chr19:6338825-6338961
Metapld, chr2:71511411-71511560
Mrps2, chr2:28468820-28468950
Mtfr1l, chr4:134530679-134530789
Ndufs2, chr1: 171238286-171238372
Neu1, chr17:34934002-34934185
Npdc1, chr2:25408904-25409001
Nrli3, chr1:171217313-171217430
Nxf1, chr19:8766796-8766833
Pde9a, chr17:31460178-31460261
Phc1, chr6:122322324-122322472
Phykpl, chr11:51593915-51594141
Rnf167, chr11:70649911-70650017
Rnpepl1, chr1:92917633-92917746
Rnpepl1, chr1:92917146-92917746
Rpl3, chr15:80081614-80081783
Rsrp1, chr4:134926734-134926818
Saal1, chr7:46701780-46701961
Selenbp1, chr3:94937954-94938075
Sirt7, chr11:120620646-120620883
Slc25a11, chr11:70645349-70645440
Slc35f6, chr5:30655887-30656100
Slc6a9, chr4:117864753-117864888
Spsb3, chr17:24890832-24890935
Spsb3, chr17:24891010-24891136
Srsf5, chr12:80949094-80949168
Srsf6, chr2:162933425-162933550
Timm44, chr8:4266555-4266680
Tmem208, chr8:105328607-105328692
Ugdh, chr5:65422634-65422782
Uros, chr7:133691085-133691166
Wbp1, chr6:83120771-83120873
Zfp26, chr9:20444893-20444985

### [Test Example 2]

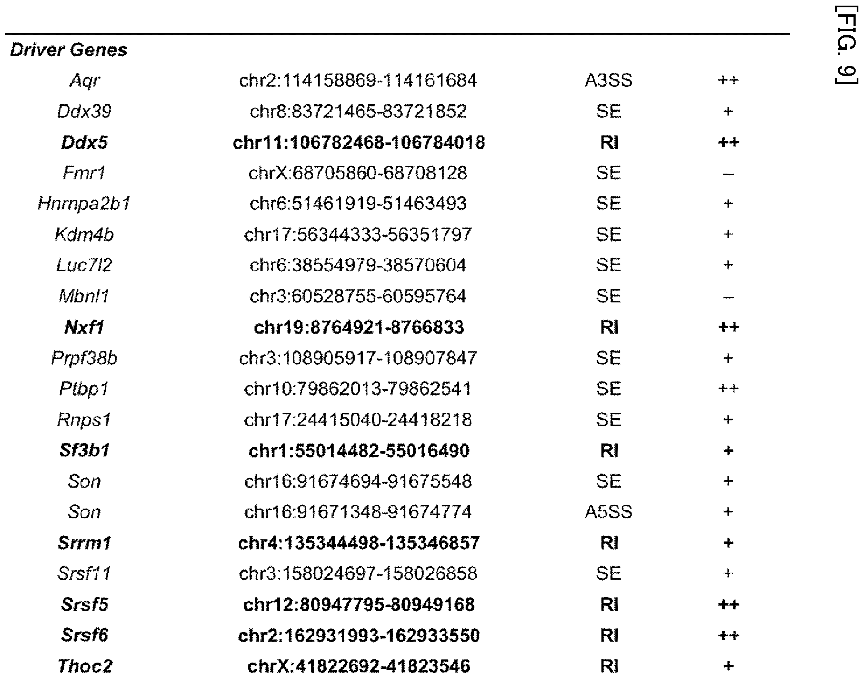

In Test Example 1, the influence of continuous feeding with the additive-containing feed on splicing was analyzed for the Cyp27a1 gene and the like. In Test Example 2, the effect of Juzen-taiho-to was further investigated for loci where alternative splicing was not properly performed according to the results of focused analysis of driver genes. Specifically, in Test Example 2, it was investigated whether or not splicing was performed in a normal manner for 20 loci serving as upstream genes out of 368 loci in genes in the liver (liver 368 loci). FIG. 9 is an explanatory diagram of upstream genes in the liver of a subject. "Driver Genes" in FIG. 9 refer to upstream genes.

As described above, upstream genes show dominant behavior over many downstream genes, and hence, when their splicing is not performed in a normal manner, splicing is not performed in a normal manner in the downstream genes, either.

In each of the upstream genes except for Mbnl1 and Fmr1 out of the upstream genes (Driver Genes) shown in FIG. 9, i.e., Aqr, Ddx39, Ddx5, Fmr1, Hnmpa2b1, Kdm4b, Luc7l2, Mbnl1, Nxf1, Prpf38b, Ptbp1, Rnps1, Sf3b1, Son(2), Srrm1, Srsf11, Srsf5, Srsf6, and Thoc2, the following fact was recognized: feeding of klotho mice with the additive-containing feed recovered the splicing pattern from an aged type to a healthy type. That is, it was recognized that splicing was caused to be performed in a normal manner.

In FIG. 9, the "Recovery Level" item of a locus where the splicing pattern was found to be recovered is expressed as "+", and the "Recovery Level" item of a locus where a particularly great recovery effect was recognized is expressed as "++". For example, "chr2:114158869-114161684" written beside Aqr represents its chromosome number in mice and position on the genome. "(2)" means that splicing was recovered in different loci on the same gene.

In Test Example 2, downstream genes of the upstream genes in which recovery was found were also investigated as to whether or not splicing patterns were recovered in the same manner as in Test Example 1. As a result, it was recognized that the splicing patterns were changed in many downstream genes, that is, were changed from aged types to healthy types therein. Upstream genes show dominant behavior over downstream genes, and hence it is conceivable that the recovery of the splicing patterns in the upstream gene resulted in the recovery of the splicing patterns in the many downstream genes as well.

In particular, in the liver of the klotho mice continuously fed with the additive-containing feed, the splicing patterns of the upstream genes in 19 loci were changed from aged types to healthy types. Meanwhile, changes in splicing patterns were not found in the upstream genes in 2 loci. It is conceived that such selective appearance of the recovery effect on the upstream genes in terms of splicing pattern in the precursor mRNA may be regarded as one of the features of Juzen-taiho-to.

As described above, in this experiment, specimen data including data on precursor mRNAs of subjects was acquired from the subjects, and as shown in FIG. 3(a), FIG. 3(b), and FIG. 3(c) to FIG. 7(a), FIG. 7(b), and FIG. 7(c), the acquired specimen data was subjected to the analysis of the removal states of introns in the precursor mRNAs. In addition, subjects in which splicing patterns with large amounts of introns remaining in mRNAs even after splicing were detected were fed with Juzen-taiho-to selected from pharmaceutically acceptable substances, and as a result, it was recognized that the introns were cleaved in the mRNAs to allow the exons to be properly joined together.

In view of the foregoing, it is conceived that the introns can be cleaved in the mRNAs to allow the exons to be properly joined together by selecting and feeding Juzen-taiho-to to the subjects in which splicing patterns with large amounts of introns remaining in the mRNAs even after splicing are detected.

In addition, in Test Examples 1 and 2, it was recognized that splicing patterns were changed in 702 genes in association with the aging of klotho mice, and that, of the 702 genes, 27 genes were upstream genes. Besides, it was recognized that, in the liver, the feeding with Juzen-taiho-to exhibited the splicing pattern-recovering effect on 19 loci.

However, in different types of aging, such as Alzheimer's aging and other diseases, it is possible that changes may occur in different upstream genes. Accordingly, it is conceived that, when changes in splicing patterns in the precursor mRNAs of individual genes are analyzed by investigating the IJC and/or the SJC in splicing in more detail, it can be identified in which gene the splicing pattern is changed for a change in healthy state, such as a disease, as well as aging.

Meanwhile, it is conceived that, for the locus of each gene, a substance that recovers the splicing pattern in the locus, or a combination of such substances can also be identified. For example, in Test Example 2, the splicing patterns of upstream genes in 19 loci were shown to be recovered by Juzen-taiho-to for the liver in the aging of klotho mice, whereas recovery was not achieved by Juzen-taiho-to in two loci Mbnl1 and Fmr1.

When a substance that recovers the splicing patterns for those two loci, Mbnl1 and Fmr1, can be identified by testing various substances in the same manner as in Test Example 1, it is conceived that such aging- or disease-causing loci can each be normalized through feeding with a combination of the substance and Juzen-taiho-to.

When naturally aged wild-type mice and Alzheimer's aged wild-type mice were also actually fed with Juzen-taiho-to, followed by observation of gene sets that underwent changes in splicing patterns, different sets were found in a set of upstream genes in 23 loci as in the case of klotho mice. It is inferred from this fact that, also in other eukaryotes having the same kind of organs or cytoplasm as klotho mice, the ingestion of Juzen-taiho-to changes the patterns of alternative splicing in at least 23 loci related to genes in the liver.

The results of this experiment are similarly applicable to humans, which are also eukaryotes. That is, when a human ingests Juzen-taiho-to, a splicing pattern in the liver can be recovered from an aged type to a healthy type. The Juzen-taiho-to to be used is, for example, a solid, such as powder, or a liquid substance obtained by dissolving the substance in a liquid, a solid substance obtained by solidifying the substance, or any other product obtained by making the substance ingestible as a formulation.

In addition, analysis for determining what substance has a splicing pattern-recovering effect on which loci of genes can be performed by performing analysis in the same manner as in Test Example 1 with substances ingestible for humans and animals, such as Kampo medicines other than Juzen-taiho-to (e.g., Hochuekkito) and drugs.

In addition, in this experiment, the analysis of mRNAs was performed with the liver. However, an upstream gene that has been changed to an aged type and a substance for changing the upstream gene can be identified for, for example, a tissue other than the liver, such as the health of skin, or blood. Accordingly, the recovery effects of Kampo medicines, other traditional medicines, supplements, or other materials on the aging- or disease-causing splicing patterns in precursor mRNAs can be associated as normalization of the splicing patterns of upstream genes analyzed from specimen data on the blood of an individual human or animal. In addition, it is conceived that barcoding with such association relationships allows an appropriate drug to be prescribed to a specific patient or animal.

That is, the results of this experiment are also applicable to precision medicine (system of performing medication in accordance with an individual's disease or aging).

In view of the foregoing, an information processing apparatus suitable for precision medicine and the like according to an exemplary embodiment is described below.

### [Configuration of Information Processing Apparatus]

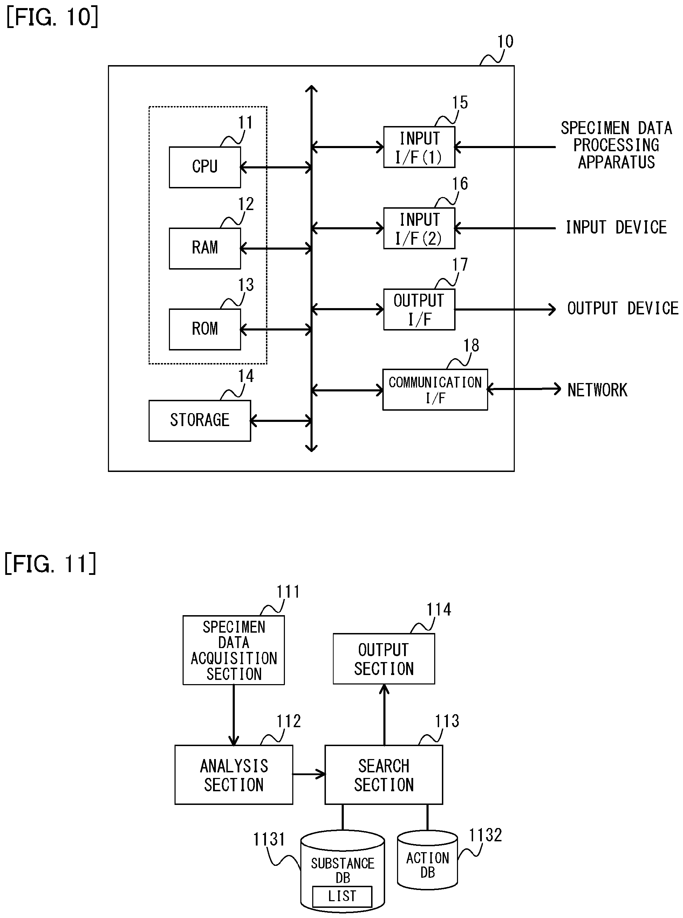

FIG. 10 is a hardware configuration diagram of an information processing apparatus 10 according to this embodiment. The information processing apparatus 10 includes a central processing unit (CPU) 11, a read only memory (ROM) 12, and a random access memory (RAM) 13, which serve as a computer, a storage 14, an input I/F (I/F is an abbreviation of interface) (1) 15, an input I/F (2) 16, an output I/F 17, and a communication I/F 18.

The CPU 11 controls the operation of the entirety of the information processing apparatus 10 by executing a computer program stored in the ROM 12 through use of the RAM 13 as a work area. The storage 14 is a mass storage device, such as a hard disk drive (HDD) or a solid state drive (SSD). The storage 14 stores a computer program and data required for the execution thereof, and also provides areas for storing various databases (abbreviated as DB) to be described later. A specimen data processing apparatus for holding analysis results of blood collected from a subject to be examined is connected to the input I/F (1) 15. An input device, such as a keyboard, a mouse, or a touch panel, is connected to the input I/F (2) 16. An output device, such as a display, a printer, a speaker, or an external storage device, is connected to the output I/F 17. A communication network, such as the Internet, is connected to the communication I/F 18.

In the information processing apparatus 10, the CPU 11 executes the computer program stored in the storage 14 to analyze a precursor mRNA of the subject to be examined (human), to thereby realize various functional blocks for facilitating precision medicine. A configuration example of those functional blocks is illustrated in FIG. 11. As illustrated in FIG. 11, the information processing apparatus 10 includes a specimen data acquisition section 111, an analysis section 112, a search section 113, and an output section 114, and also includes a substance DB 1131 and an action DB 1132.

The substance DB 1131 is a DB in which information on a pharmaceutically acceptable substance for bringing a splicing pattern (second splicing pattern to be described later) that is known in advance through an experiment or the like to be changed closer to a splicing pattern before the change (first splicing pattern to be described later) is stored for each second splicing pattern.

The pharmaceutically acceptable substance is as described above in this experiment. The substance is generally an ingestible substance, and it may be appropriate that one ingestible substance be associated with another ingestible substance not compatible therewith so that the other substance can also be read out at the time of the retrieval of the one substance. With this configuration, for example, the following information can be obtained: one substance brings the second splicing pattern closer to the first splicing pattern, but the other substance exhibits an opposite effect.

In the substance DB 1131, for second splicing patterns that are frequently searched for, a list that records information on the substances for bringing the second splicing patterns closer to the first splicing pattern (substance list) is stored. Accordingly, when a second splicing pattern is on the list, information on the pharmaceutically acceptable substance (information on the above-mentioned one substance and/or other substance) can be rapidly retrieved.

In the action DB 1132, information on actions for bringing the second splicing pattern closer to the first splicing pattern before the change, such as resting for a predetermined period of time after the ingestion of the substance identified in the substance DB 1131, performing a characteristic exercise, and relaxing, in other words, information on non-ingestion (-eating) measures is stored with keywords including the second splicing pattern.

The specimen data acquisition section 111 acquires, from a subject (human), specimen data including data on a precursor mRNA of the subject. It may be appropriate to acquire identification information for identifying each living environment from identical subjects placed in different living environments and specimen data from each of the subjects. In the latter case, when the identification information is stored and associated with analysis results of the analysis section 112 to be described later, analysis results of mRNAs in each living environment can be obtained.

In this embodiment, data obtained by analyzing blood collected from the subject with the specimen data processing apparatus is used as the specimen data. However, the specimen data is not limited thereto, and analysis data on blood collected at a blood collection facility or a hospital may be acquired through the specimen data processing apparatus. The specimen data includes information representing the states of the internal organs and cells of the subject.

The analysis section 112 quantitatively analyzes the removal state of an intron in the precursor mRNA (information on the result of alternative splicing) for the specimen data acquired in the specimen data acquisition section 111, to thereby detect the presence or absence of a second splicing pattern having the possibility of deriving a gene expression pattern different from that of a first splicing pattern that derives a predetermined gene expression pattern. As described in the above-mentioned experiment, the "removal state" includes a state in which an intron to be normally removed remains. The term "quantitatively" means that the above-mentioned state can be expressed as, for example, a numerical value or mass. In this embodiment, for the sake of convenience, the presence or absence of the second splicing pattern in an upstream gene out of regulators of splicing in the specimen data acquired from the subject to be examined is detected.

The first splicing pattern is a splicing pattern serving as an object of comparison in the analysis of the state of the precursor mRNA of the subject to be examined, and refers to, for example, a splicing pattern that derives a gene expression pattern obtained through analysis of a large number of specimen data acquired from healthy humans younger than about 20 years old. However, it is also appropriate to: perform the above-mentioned analysis for each of specimen data acquired a plurality of times from a single subject to be examined to accumulate respective pieces of information on the results of alternative splicing in a time series manner; and adopt, out of the accumulated respective pieces of information on the results, information on the result of alternative splicing that derived the result information most similar to information on the result of alternative splicing by a eukaryote serving as a reference (e.g., a set of the above-mentioned healthy humans) as the first splicing pattern for the subject to be examined. In this case, the first splicing pattern for the subject to be examined is stored in a predetermined area of the storage 14, and is read out as appropriate.

The analysis section 112 may be realized using the above-mentioned "rMATS" and R language. Accordingly, the analysis section 112 may be operated as visualization means configured to visualize information on the result of alternative splicing.

The search section 113 retrieves, when the second splicing pattern is detected, information on a pharmaceutically acceptable substance for bringing the second splicing pattern closer to the first splicing pattern from the substance DB 1131. At that time, when the action DB 1132 stores information on actions, such information is also retrieved.

The output section 114 outputs those kinds of retrieved information to the output device as information specific to the subject to be examined. The output section 114 may output only the information on the result of alternative splicing.

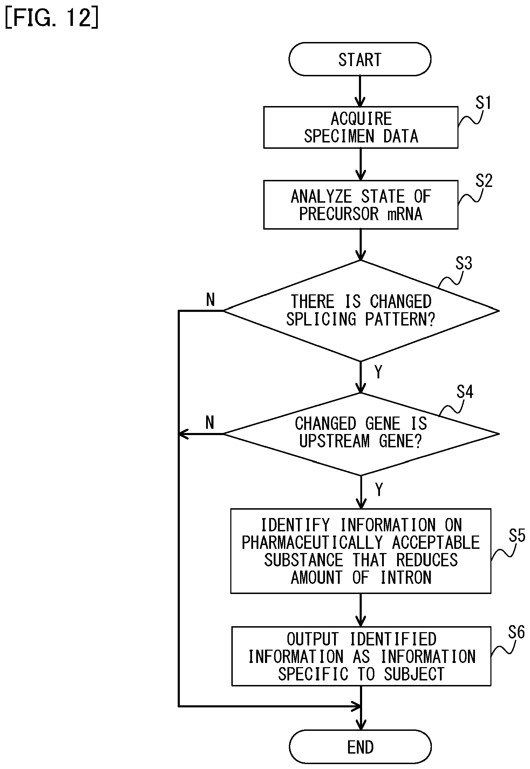

An example of the procedure of processing executed in the information processing apparatus 10 configured as described above is illustrated in FIG. 12. Referring to FIG. 12, the information processing apparatus 10 first acquires specimen data on a subject (human) (Step S1). The specimen data is, for example, analysis data on blood collected from the subject. The information processing apparatus 10 then analyzes the state of a precursor mRNA (Step S2). Specifically, quantitative analysis is performed as shown in FIGS. 3 to FIGS. 7. After that, the information processing apparatus 10 determines whether there is a splicing pattern (second splicing pattern) changed with respect to a splicing pattern serving as a reference (first splicing pattern) (Step S3). When the second splicing pattern is present (Step S3: Y), it is determined whether the changed splicing pattern belongs to an upstream gene (Step S4). In the case of an upstream gene (Step S4: Y), information on a pharmaceutically acceptable substance that reduces the amount of an intron is identified (Step S5). In this step, when information on a related action exists, such information is also identified. Then, those kinds of identified information are output to the output device as information specific to the subject (Step S6), and the processing is ended.

When it is determined in Step S3 that there is no changed splicing pattern (Step S3: N) or when it is determined in Step S4 that the changed splicing pattern does not belong to an upstream gene (Step S4: N), the processing is immediately ended.

Through execution of such procedure, when there is a change in a splicing pattern at the time of the acquisition of specimen data on a subject, information on a substance appropriate for recovery or the like is output as information specific to the subject, and hence, for example, the proposal of information on a measure in the event that the subject is in an aged state can be facilitated.

### [Other Discussions based on this Experiment]

It has hitherto been known that an overall change in alternative splicing is induced by cancer. Similarly, it was recognized through this experiment that an undesired state, such as aging, also induces an overall change in alternative splicing.

Changes in splicing patterns due to aging in alternative splicing of many genes are caused by changes in expression of splicing factors. Alternative splicing is tied to transcription, and hence a mechanism that controls transcription may also control alternative splicing.

For example, chromatin components, and epigenetic factors, such as factors related to histone modification and DNA methylation, not only control transcription, but also regulate splicing. In this experiment, the aging of genes was detected through the removal states of introns in alternative splicing, and it was recognized that the removal states of introns were recovered to healthy types by giving eukaryotes a pharmaceutically acceptable substance.

However, whether a gene is aged, and whether an aged gene is returned to a healthy type by Juzen-taiho-to or the like may be recognized by using, in place of the removal states of introns, chromatin components, and epigenetic factors, such as factors related to histone modification and DNA methylation.

For example, after the aging of a gene has been recognized on the basis of a low level of DNA methylation, and a pharmaceutically acceptable substance, such as Juzen-taiho-to, has been given, whether or not the gene has been returned to a healthy type may be found out by detecting whether or not the DNA methylation has been normalized. Further theoretical background on the applicability of such DNA methylation and the like to finding out whether a gene is of an aged type or a healthy type is described below.

The above-mentioned epigenetic factors influence the elongation rate of Pol II, and by extension, influence splicing patterns. For example, standard histone-depleted human cells show changes in transcription and splicing that are often observed in aged tissues. The depletion of histones increases the elongation rate of Pol II to cause the exclusion of exons, which agrees with the "kinetic model" of cotranscriptional splicing. In the case of epigenetic histone modification, recent investigations show that the depletion of KDM5B, which is a demethylase specific for H3K4me3/2, causes a reduction in Pol II promoter occupancy, which then induces slow Pol II elongation, to thereby influence the expression of alternatively spliced exons in embryonic stem cells.

Those data suggest the main importance of the regulation of a transcription elongation rate with respect to the exclusion and inclusion of exons. It is presumed that a change in transcription elongation rate originally induced by a change in histone modification is involved to some extent in a wide range of aging-associated changes in SE patterns (exon exclusion removal or exon inclusion) and recovery therefrom after Juzen-taiho-to treatment.

There are two models for controlling the selection of splice sites, i.e., a kinetic model and a recruitment model, each of which includes the transcription elongation rate. In the "kinetic model", alternative splicing is influenced by influencing the transcription elongation rate, that is, the pace at which splice sites and regulatory sequences appear in a new mRNA during transcription.

In particular, when elongation is fast, a stronger splice site can be utilized, and hence the use of the stronger splice site, rather than a weaker (upstream) splice site, is promoted, resulting in the removal of an exon. Meanwhile, when elongation is slow, the replenishment (recruitment) of standard splicing factors to a weaker splice site on the upstream side is promoted before a stronger splice site on the downstream side can be synthesized, resulting in the inclusion of an exon.

In the "recruitment model", other players, such as inhibitory and stimulatory splicing factors, can bind to a splice site within a specific time frame set on the basis of the pace of transcription, resulting in exon inclusion or exon exclusion.

Surprisingly, it was observed after the administration of Juzen-taiho-to that 97.2% of genetic loci subjected to intron retention had been recovered to healthy types. This shows or strongly suggests the involvement of DNA methylation and RNA Pol II elongation in this process. When the level of DNA methylation was low, the binding of methyl CpG binding protein 2 (MeCP2) was reduced in the vicinity of a splice junction in DNA encoding an unspliced intron. As a result of this, the recruitment of splicing promoting factors, such as TRA2b (SRSF10) and Srsf family members, is reduced to cause intron retention for the corresponding mRNA.

Further, a reduction in MeCP2 level is related to a reduced rate of RNA Pol II elongation at a genomic position corresponding to intron retention. The reduced rate of Pol II elongation contributes to increasing the recruitment of splicing repressors, leading to a further increase in intron retention. It may be said from those data that the linking together of the above-mentioned two models has demonstrated the causal role of reduced DNA methylation in the promotion of intron retention.

Genes encoding certain Srsf family members (srsf5, 6, and 11) were detected as upstream genes whose splicing patterns changed with aging, but the introns of Srsf5 and Srsf6 are retained even during aging. The activity of each of those genes may be reduced by a monitoring mechanism, such as nonsense-mediated decay. Consequently, a contribution may be made to the accumulation of intron retention through loss of MeCP2 resulting from a reduction in recruitment of those factors.

It may be said that the recovery of the splicing patterns in many genes after the ingestion of Juzen-taiho-to is caused by the normalization of DNA methylation and the acceleration of the transcription elongation rate promoted by the recovery of the Srsf family members from intron retention.

Such theoretical background as described above also shows the applicability of DNA methylation and the like to finding out whether a gene is of an aged type or a healthy type.

As described above, upstream genes dominate (control) the splicing of downstream genes, the ingestion of a substance such as Juzen-taiho-to returns an upstream gene from an aged type to a healthy type, and the upstream gene returned to a healthy type returns a downstream gene changed into an aged type to a healthy type. However, the possibility that the substance such as Juzen-taiho-to itself has an action of returning a downstream gene to a healthy type by directly acting thereon is also conceivable.

That is, it cannot be asserted that the cause of the conversion of a downstream gene from an aged type to a healthy type is limited to one derived from an upstream gene. Accordingly, although, in the foregoing description, upstream genes were targeted as genes converted to healthy types in alternative splicing, and were subjected to analysis for determining a pharmaceutically acceptable substance for returning a gene from an aged type to a healthy type, the substance for returning a gene from an aged type to a healthy type can be determined with higher resolution or higher accuracy by similarly analyzing downstream genes as well.

Specifically, 350 or more genes including upstream genes and downstream genes are changed in the liver, and when all these genes are used as analysis objects, the effect of, for example, the ingestion of a pharmaceutically acceptable substance or a change in degree of aging can be more accurately detected. However, it takes a great deal of time and effort to analyze all those genes, and hence the analysis of upstream genes is advantageous in efficiently detecting the effect of, for example, the ingestion of a substance or a change in degree of aging.

In the foregoing description, 97.2% of the mRNAs exhibiting intron retention in their splicing patterns were found to undergo the recovery of the splicing patterns, and hence mRNAs exhibiting intron retention in their splicing patterns were mainly used as main objects. However, the present invention is not limited thereto, and is also applicable to other splicing patterns, such as the above-mentioned Skipped Exon (SE) pattern, Alternative 5' Splice Site (A5SS) pattern, Alternative 3' Splice Site (A3SS) pattern, and Mutually eXclusive Exons (MXE).

As described above, among the genes in the liver, changes in alternative splicing are found in 368 loci between the klotho mice repeatedly fed with the additive-containing feed and the klotho mice fed only with the normal feed. 88.3% of those (325 loci) are the recovery of a splicing pattern from an aged type to a healthy type. This also clearly shows that the recovery of a splicing pattern from an aged type to a healthy type is not limited to intron retention, but also occurs in other splicing patterns.

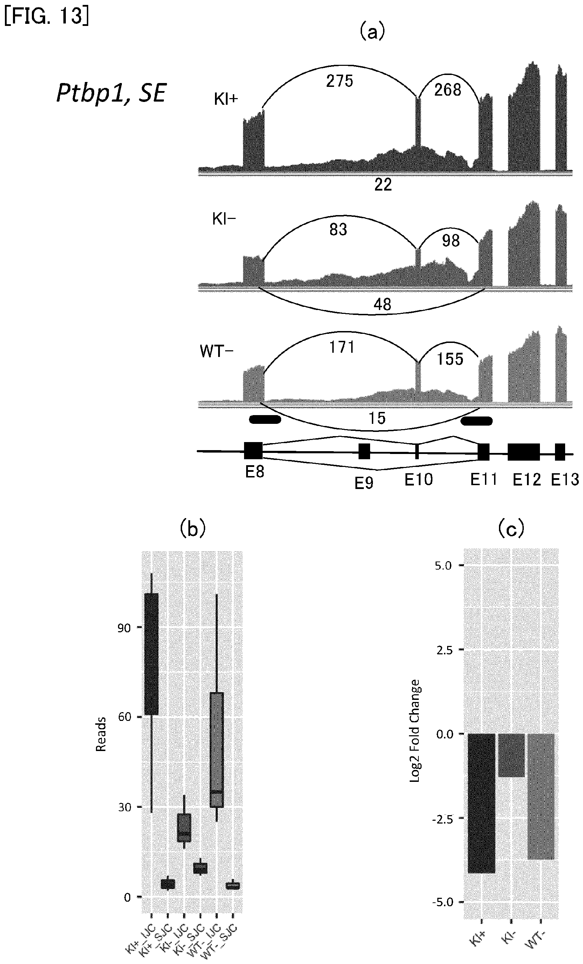

FIG. 13(a) to FIG. 13(c) are explanatory diagrams of the Skipped Exon (SE) splicing pattern, which is illustrated in the second row of FIG. 1, in the upstream gene Ptbp1 in Test Example 1. In FIG. 13(a) to FIG. 13(c), Kl+ and Kl- represent, like KL+ and KL- in FIG. 3(a) and the like, klotho mice fed with the additive-containing feed of Juzen-taiho-to and klotho mice not fed with the additive-containing feed of Juzen-taiho-to, respectively. In addition, the other symbols, such as WT, are identical to the symbols in FIG. 3(a) and the like.

Referring to FIG. 13(a), exon E9 is skipped, indicating that the splicing pattern is SE. Referring to FIG. 13(b), the pattern of read values of the klotho mice (K1-) not fed with the additive-containing feed of Juzen-taiho-to is different from the patterns of read values of the klotho mice (Kl+) fed with the additive-containing feed of Juzen-taiho-to and the wild-type mice (WT-) not fed with the additive-containing feed of Juzen-taiho-to.

In addition, as shown in FIG. 13(c), in terms of fold change value, namely log₂IJC/SJC value, Kl+ and WT- have similar values, but the value of Kl- is about 1/3, which is a clearly small value. This shows that feeding with the additive-containing feed of Juzen-taiho-to recovered the SE splicing pattern from an aged type to a healthy type in the upstream gene Ptbp1 of klotho mice.

Accordingly, FIG. 13(a) to FIG. 13(c) also clearly show that the recovery of a splicing pattern from an aged type to a healthy type is not limited to intron retention, but also occurs in other splicing patterns.

### <Second Embodiment>

Next, a second embodiment of the present invention is described. In the second embodiment, an analysis example of a biological state for the human body is described. That is, description is made of an example in which the state of the human body serving as a biological state is analyzed on the basis of measurement results of a measurement object. Examples of the measurement object include biosignals emitted from the body through biological phenomena, such as a heartbeat, a brain wave, a pulse, breathing, and perspiration, but other objects may also be used. In the second embodiment, with gene expression being taken as an example, the biological state of the human body was analyzed through use of the concept of "presymptomatic disease".

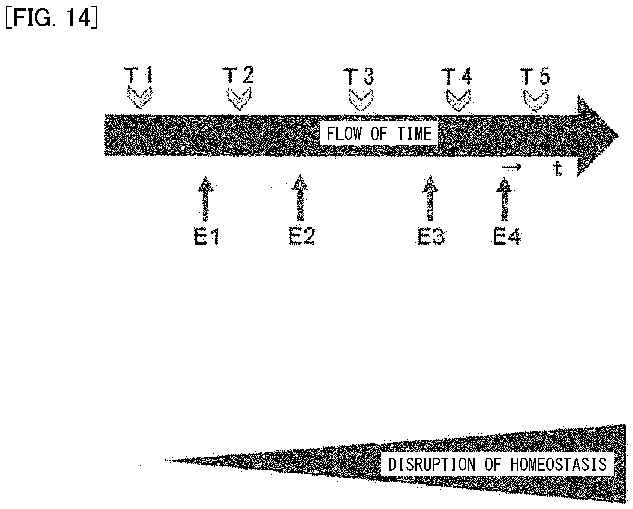

FIG. 14 is an explanatory diagram of the concept of presymptomatic disease. In FIG. 14, the flow of time is indicated by the direction of an arrow. The direction from left to right across FIG. 14 represents the direction of time passage. A time point close to the left end of FIG. 14 represented by T1 represents a healthy state, and E1 represents the starting time point of a presymptomatic disease due to, for example, a slight change in metabolism.

As used herein, the term "presymptomatic disease" refers to a state in which an organism has some abnormality from a healthy state owing to a slight change in metabolism or any other factor but the influence thereof is not manifested. That is, the presymptomatic disease may also be said to be a state between health and a disease. For the sake of convenience, such state is sometimes referred to as "presymptomatic disease state".

The presymptomatic disease state is not diagnosed as a disease by existing criteria, but has a significantly high risk of developing some disease. In addition, as illustrated in FIG. 14, the disruption of homeostasis is increased with the passage of time.

After the occurrence of the presymptomatic disease state, the intron retention described in the first embodiment appears. This is represented by E2 in FIG. 14. After that, the appearance of the intron retention at E2 influences gene expression (protein expression) to be manifested at E3. As a result of the occurrence of the change in protein expression, a pathological abnormal finding appears at E4. As a result of the appearance of the pathological abnormal finding as just described, the human body is led to a disease or death.

In addition, in FIG. 14, T2 represents a time point between the starting time point E1 of the presymptomatic disease and the appearance time point E2 of the intron retention. In the following description, an example in which a genetic locus where the intron retention appears at a time point after E2 is identified is described. That is, when the intron retention has yet to appear but the occurrence of, for example, a change in metabolism can be detected at T2 between E1 and E2, the presymptomatic disease state can be detected even at the time point T2. The presymptomatic disease state is desirably detected as early as possible.

In FIG. 14, T3 represents a time point between the appearance of the intron retention and the manifestation of the change in protein expression, and T4 represents a time point between the manifestation of the change in protein expression and the appearance of the pathological abnormal finding. The time points T3 and T4 are both before the appearance of the pathological finding, and hence, when a genetic locus in which the intron retention has appeared can be identified at any one of these time points, the presymptomatic disease can be detected. In addition, when corresponding treatment, such as JTT administration, is performed in response to the identified intron retention, the human body can even be returned from the presymptomatic disease state to a healthy state before the appearance of the pathological abnormal finding.

In presymptomatic disease research, there is also known a mathematical theory with a focus on the fluctuation of biosignals called the "dynamical network biomarker theory (DNB theory)." In the DNB theory, actual data is analyzed with a practically simplified index to scientifically detect a presymptomatic disease for metabolic syndrome. In the DNB theory, a time point at which the fluctuation of some mutually related biosignals is significantly increased immediately before transition from a healthy state to a disease state is defined as a presymptomatic disease state, and hence the presymptomatic disease can be quantitatively and directly detected through analysis of biosignal data.

In particular, under a state in which homeostasis is maintained, that is, a normal (healthy) state, analysis of the expression amounts of genes according to the DNB theory showed the presence of a state having low energy and showing high robustness and high resilience. In addition, under the presymptomatic disease state, that is, a state before disease, a state having high energy and showing the occurrence of fluctuation was observed in the expression of genes. This state is a state showing low robustness and low resilience.

In addition, it was recognized that the normal state and the presymptomatic disease state were reversible, and it was possible to return to the normal state from the presymptomatic disease state through appropriate treatment. Meanwhile, when left untreated, the presymptomatic disease state became an abnormal (disease) state, and under this state, a state having low energy and showing high robustness and high resilience was established in the expression amounts of genes. In addition, the presymptomatic disease state and the abnormal state were irreversible, and once established, the abnormal state was impossible to return to the presymptomatic disease state.

In addition, in this research, mice that were to spontaneously develop metabolic syndrome (TSOD mice) were reared, and the expression amounts of genes in adipose tissue were comprehensively measured by a microarray method every other week from 3 weeks old to 7 weeks old. Next, data analysis based on the DNB theory was performed to investigate whether there was a time point showing an increase in fluctuation during the measurement period, and as a result, it was revealed that the fluctuations of the expression amounts of 147 genes were significantly increased at the time point of 5 weeks old before the mice developed metabolic syndrome.

As described above, in the above-mentioned research, the presymptomatic disease state was quantitatively detected according to the DNB theory. On the other hand, in the second embodiment, instead of analysis based on a theory, an approach involving detecting the presymptomatic disease state through measurement of intron retention in m-RNA was performed, and besides, it was made possible to return the presymptomatic disease state to the normal state. In particular, in the second embodiment, a state having intron retention caused in a genetic locus is defined as the presymptomatic disease state, and the genetic locus having intron retention caused therein is identified. Then, in response to the genetic locus, a measure such as JTT administration was performed as in the first embodiment to achieve recovery from intron retention, consequently returning the presymptomatic disease state to the healthy state.

The provision of a specific technique for not only detecting a presymptomatic disease state, but also recovering the presymptomatic disease state as described above has not been known heretofore, and has been made possible for the first time by the present invention. In the following description, a state in which intron retention has occurred as a result of a failure to perform alternative splicing in a normal manner is referred to as "presymptomatic disease-type splicing pattern" for the sake of convenience.

In the second embodiment, intron retention in klotho mice was measured in the same manner as in the first embodiment, and the characteristics of genes that recovered from the presymptomatic disease-type splicing pattern and genes that did not recover were investigated.

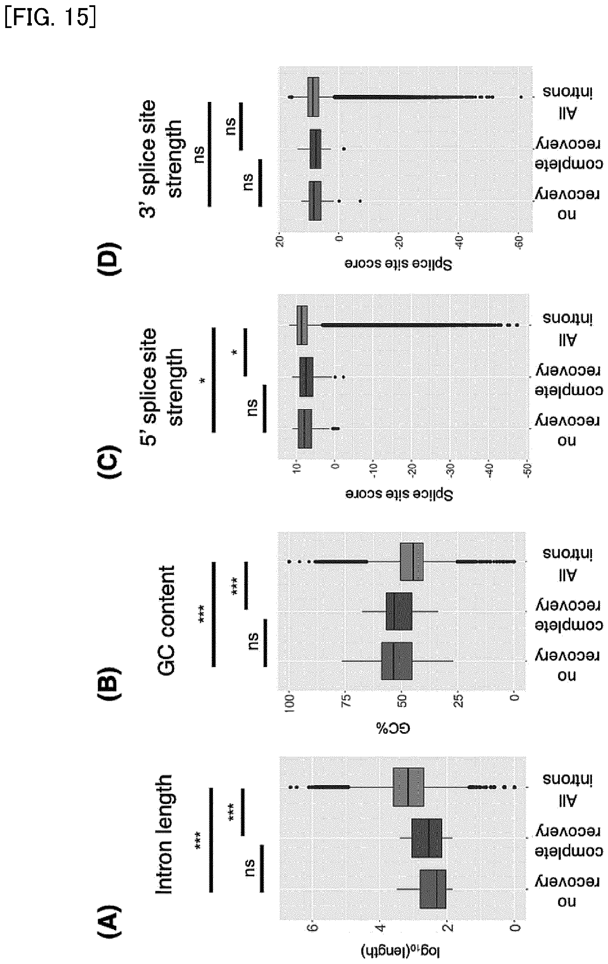

Specifically, characteristics were measured of introns of genes shown in FIG. 8, i.e., 70 "complete recovery" genes, 120 "no recovery" genes, and all other genes (about 250,000 genetic loci) expressed in the liver. Graphs showing the measurement results of the characteristics of the introns of the genes are shown in FIG. 15(A) to FIG. 15(D).

In FIG. 15(A), "intron length" represents the length of introns, and the vertical axis represents the common logarithmic value of the intron length (log₁₀(length)). In FIG. 15(B), "GC content" represents a GC content (ratio of guanine and cytosine among the four bases in a DNA molecule, GC%). In FIG. 15(C), "5' splice site strength" represents the strength of a 5' splice site. The strength may be represented as a splice site score on the vertical axis in FIG. 15(C). In FIG. 15(D), the strength and score of a 3' splice site are similarly shown.

As shown in FIG. 15(A), the lengths of the introns in the "complete recovery" and "no recovery" genetic loci were markedly shorter than the lengths of the introns in all the other genes. However, there was no significant difference between the intron lengths of the "complete recovery" genetic loci and "no recovery" genetic loci. This suggests that, in a recovery process related to JTT, the "complete recovery" genetic loci were not selectively identified from the "no recovery" genetic loci.

Meanwhile, the GC contents of the introns for the "complete recovery" and "no recovery" groups were significantly higher than the GC content of all the other liver-related introns (FIG. 15(B)). In addition, there is no significant difference in this regard between the "complete recovery" introns and "no recovery" introns, suggesting that the GC content is not related to the recovery state of RI genetic loci.

Next, the strengths of the 5' and 3' splice sites were investigated by calculating the scores of those splice sites through use of software MaxEntScan.

The 5' splice sites of the introns of the "complete recovery" and "no recovery" genetic loci were significantly weaker, though to a slight degree, than the splice sites of all the other liver-related introns (FIG. 15(C)), but such difference was not found for the 3' splice sites (FIG. 15(D)).

Those characteristics of the introns of intron retention (RI) genetic loci are observed in three other organs (see FIG. 15(A) to FIG. 15(D)), which agrees with hitherto reported data (Braunschweig et al., 2014).

Accordingly, genetic loci having a short intron length, a high GC content, and a low splicing score at the 5' site tend to retain introns.

In addition, as shown in FIG. 15(A) to FIG. 15(D), RI genetic loci have distinguishing characteristics. FIG. 15(A) to FIG. 15(D) show box plots of various data measured for introns. FIG. 15(A) shows intron lengths, FIG. 15(B) shows the GC percentage in intron sequences, FIG. 15(C) shows the strength score of the 5' splice site, and FIG. 15(D) shows the strength score of the 3' splice site.

FIG. 15(A) to FIG. 15(D) show comparisons among the three groups of introns, namely "no recovery" ("no recovery" in the figures), "complete recovery" ("complete recovery" in the figures), and all introns from liver-expressed genes (254,005 genetic loci) ("All introns" in the figures). The results in FIG. 15(A) to FIG. 15(D) were tested by t-test. Significance levels are *P≤0.05 and ***P≤0.001, and ns stands for not significant.

On the basis of those results, genetic loci having a possibility of causing intron retention can be narrowed down. In particular, on the basis of the results of FIG. 15(A), FIG. 15(B), and FIG. 15(C), introns having a short intron length, a high GC content, and a weak 5' splice site are highly liable to cause intron retention, and hence these indicators can be used to narrow down genetic loci in which intron retention is liable to occur.

For example, it is known that a single gene contains generally 10 to 30 exons with an average of about 20, and that the human body contains about 20,000 to about 30,000 genes. Thus, 400,000 to 600,000 kinds of exons exist in the human body, and hence it is extremely difficult to determine whether or not intron retention may occur for these exons.

Meanwhile, in the second embodiment, as described above, exons that may cause intron retention can be narrowed down with the criteria of introns having a short intron length, a high GC content, and a weak 5' splice site. As a result, out of the 400,000 to 600,000 kinds of genetic loci, exons that may presumably cause intron retention can be narrowed down to about a little less than 10,000. As a result, the detection of intron retention can be performed with the narrowed down genetic loci.

Genetic loci having a possibility of causing intron retention have been described, which can be used to narrow down genetic loci that can be recovered to some extent from intron retention. When a little less than 10,000 genetic loci thus identified are subjected to a presymptomatic disease test with a microarray for a presymptomatic disease test, the time and effort involved in the presymptomatic disease test can be dramatically reduced. The microarray for a presymptomatic disease test is a custom microarray for a test, for performing a test for intron retention on at least some of the genetic loci narrowed down as described above.

For genetic loci, data in healthy states are already known. The microarray for a presymptomatic disease test includes: a reference data storage section storing reference data on at least part of the above-mentioned narrowed down genetic loci in healthy states; a body fluid storage section; a genetic locus information detection section; and an analysis section. When the state of a presymptomatic disease is detected with the microarray for a presymptomatic disease test, a body fluid, such as blood, collected from the human body is stored in the body fluid storage section. The genetic locus information detection section detects, from the body fluid stored in the body fluid storage section, information on genetic loci therein. The analysis section analyzes the genetic locus information detected in the genetic locus information detection section by comparing the genetic locus information and reference data on the genetic loci narrowed down as described above, to thereby identify genetic loci having intron retention caused therein.

When the genetic loci having intron retention caused therein are identified as described above through use of the microarray for a presymptomatic disease test, a measure such as JTT administration can be performed in accordance with the identified genetic loci.

In actuality, the exons used in the first embodiment, i.e., all of the 70 "complete recovery" genes shown in FIG. 8 correspond to exons that may cause intron retention by the criteria of "introns having a short intron length, a high GC content, and a weak 5' splice site."

In addition, it was possible to perform a presymptomatic disease test by investigating the presence or absence of intron retention as in the first embodiment with respect to the 70 genes serving as exons narrowed down by the above-mentioned criteria, and performing comparisons to exons in healthy states.

As a result, the presymptomatic disease state can be returned to the healthy state through recovery from intron retention. As described above, according to the second embodiment, there is provided a specific technique for not only quantitatively detecting a presymptomatic disease state, but also achieving recovery from the presymptomatic disease state. In addition, the presymptomatic disease state can be detected and recovered to the healthy state for some genetic loci through application of the first embodiment as described above, and thus it has been verified that there can be produced a custom microarray for a test, for performing a test for intron retention, by storing information on the above-mentioned 10,000 narrowed down genetic loci.

In the foregoing description, 70 genes recognized to have undergone "complete recovery" in the liver as shown in FIG. 8 have been described. However, the present invention is not limited to the 70 genes, and is applicable to all genes that may cause intron retention.

The present invention has been described by way of the first embodiment and the second embodiment, but the present invention may also be carried out by executing the following processing. That is, a computer program for realizing the functions of the above-mentioned information processing apparatus is supplied to a computer or a system including the computer, via a network or any of various storage media. Then, in the processing, the computer reads out and executes the computer program. In this case, the computer program and a recording medium storing the computer program are included in the present invention.

## Claims

1. An analysis method for a precursor mRNA, comprising:
quantitatively analyzing a removal state of an intron in a precursor mRNA of a eukaryote, to thereby detect presence or absence of a second splicing pattern having a possibility of deriving a gene expression pattern different from that of a first splicing pattern that derives a gene expression pattern serving as a reference; and
when the second splicing pattern is present, identifying, from a pre-made substance list, information on a pharmaceutically acceptable substance for bringing the second splicing pattern closer to the first splicing pattern, and outputting the identified information as information specific to the eukaryote.

2. The analysis method according to claim 1, wherein the substance is a Kampo medicine or a liquid substance having dissolved therein a Kampo medicine.

3. The analysis method according to claim 2, wherein the liquid substance is Juzen-taiho-to.

4. An information processing apparatus, comprising:
acquisition means configured to acquire, from a eukaryote, specimen data including data on a precursor mRNA of the eukaryote;
analysis means configured to analyze a removal state of an intron in the precursor mRNA for the acquired specimen data, to thereby detect the presence or absence of a second splicing pattern having a possibility of deriving a gene expression pattern different from that of a first splicing pattern that derives a predetermined gene expression pattern;
search means configured to retrieve, when the second splicing pattern is detected, information on a pharmaceutically acceptable substance for bringing the second splicing pattern closer to the first splicing pattern from a predetermined substance database; and
output means configured to output the retrieved information as information specific to the eukaryote.

5. The information processing apparatus according to claim 4,
wherein the first splicing pattern is a splicing pattern in a gene in a healthy state, and
wherein the second splicing pattern is a splicing pattern in a gene of an aged type.

6. The information processing apparatus according to claim 4,
wherein the first splicing pattern is a splicing pattern in a gene in a healthy state, and
wherein the second splicing pattern is a splicing pattern in a gene in a presymptomatic disease state in which some abnormality is present but an influence thereof is not manifested.

7. The information processing apparatus according to any one of claims 4 to 6, wherein the analysis means is configured to detect the presence or absence of the second splicing pattern in an upstream gene that controls a downstream gene out of regulators of splicing in the acquired specimen data.

8. The information processing apparatus according to any one of claims 4 to 7, wherein the analysis means is configured to perform the analyzing for the specimen data acquired a plurality of times from a single eukaryote to: accumulate respective pieces of information on results of alternative splicing in a time series manner; and store as the first splicing pattern in the eukaryote, out of the accumulated respective pieces of information on the results, information on a result of alternative splicing that derived result information most similar to information on a result of alternative splicing by a eukaryote serving as a reference.

9. The information processing apparatus according to any one of claims 4 to 8, wherein the substance is a Kampo medicine or a liquid substance having dissolved therein a Kampo medicine.

10. The information processing apparatus according to claim 9, wherein the liquid substance is Juzen-taiho-to.

11. The information processing apparatus according to any one of claims 4 to 10, wherein the search means is configured to retrieve information on an action for bringing the second splicing pattern closer to the first splicing pattern from a predetermined action database.

12. A computer program for causing a computer to operate as the information processing apparatus of any one of claims 4 to 11.
